(19)

Europäisches
Patentamt
European
Patent Office
Office européen
des brevets

(11) **EP 1 839 572 A2**

(12) **EUROPEAN PATENT APPLICATION**

(43) Date of publication:
03.10.2007 Bulletin 2007/40

(51) Int Cl.:
*A61B 5/0488* (2006.01)   *A61B 5/04* (2006.01)
*A61B 5/00* (2006.01)   *A61B 5/00* (2006.01)

(21) Application number: 07006441.5

(22) Date of filing: 28.03.2007

(84) Designated Contracting States:
AT BE BG CH CY CZ DE DK EE ES FI FR GB GR
HU IE IS IT LI LT LU LV MC MT NL PL PT RO SE
SI SK TR
Designated Extension States:
AL BA HR MK YU

(30) Priority: 31.03.2006   JP 2006098413
31.03.2006   JP 2006099274
31.03.2006   JP 2006099319
12.05.2006   JP 2006134048

(71) Applicant: CASIO COMPUTER CO., LTD.
Shibuya-ku,
Tokyo 151-8543 (JP)

(72) Inventors:
• Kanzaki, Takashi
Hamura-shi
Tokyo 205-8555 (JP)
• Fukumura, Masaaki
Hamura-shi
Tokyo 205-8555 (JP)

(74) Representative: Grünecker, Kinkeldey,
Stockmair & Schwanhäusser
Anwaltssozietät
Maximilianstrasse 58
80538 München (DE)

(54) **Biological information measuring device and biological information measuring system**

(57) A biological information measuring system which is capable of measuring biological information simultaneously at a plurality of body sites, and of visually recognizing the biological information in real time at hand. A control device (30) respectively transmits a measurement start signal to each of myoelectric potential measuring devices (10) when a measurement start instruction is inputted from an input unit (310), and displays measurement results transmitted as needed from the respective myoelectric potential measuring devices on a display unit (330).

On the other hand, when the measurement start signals are transmitted from the control device (30), the plurality of myoelectric potential measuring devices (10) start measurement of myoelectric potentials by measuring units (110), and transmit active states of the measured muscles as measurement results to the control device (30).

**FIG.2**

EP 1 839 572 A2

**Description**

The present invention relates to a biological information measuring device and a biological information measuring system which are capable of displaying measurement results obtained by measuring biological information.

Conventionally, a biological information measuring system which measures variations in biological information such as a myoelectric potential, a pulse, a blood pressure, and a body temperature has been known. The biological information measuring system has been broadly utilized, not only as a medical device, but also with an aim to maintain good health, to grasp an exercise condition, or the like. As a biological information measuring system measuring a myoelectric potential among those, for example, a biological information measuring system which estimates a muscle state by measuring a myoelectric potential of a mounted site, and displays the estimated muscle state has been known (refer to, for example, Jpn. Pat. Appln. KOKAI Publication No. 2004-202196).

However, the measuring device disclosed in the prior art document does not have a display unit, and it is impossible to view myoelectric potential data unless a memory card storing measured myoelectric potential data is detached from the device body, and is connected to an external processing device such as a personal computer. Therefore, there is the problem that it is impossible for a user to view an active state of muscles during exercise in real time. Further, the measuring device itself is structured so as to be independent of other measuring devices, and it is difficult to measure biological information on a plurality of places of a body so as to be synchronized during the exercise. Therefore, it is impossible to find out the relationship or the relationship between one muscular movement and another muscular movement or movements.

Further, the technology disclosed in the prior art document includes a structure in which, when a display button for requesting display is pressed, a value expressing a muscle state accumulated from the start of measurement is expressed numerically. Therefore, while it is possible to display a muscle state, it is impossible to display changes over time in a muscle state according to each exercise.

An object of the present invention is to provide a biological information measuring system which is capable of measuring biological information simultaneously at a plurality of body sites, and of visually recognizing the biological information in real time.

Another object of the present invention is to provide a biological information measuring device and a biological information measuring system which are capable of displaying measurement results of several pieces of biological information in a comparable and referable format.

Yet another object of the present invention is to detect an activity interval of a predetermined site by measuring biological information at the predetermined site of a human body, and to calculate activity efficiency at each detected activity interval.

Yet another object of the present invention is to provide a biological information measuring device and a biological information measuring system which are capable of displaying measurement results of biological information in an easily-viewable display format.

In order to achieve the above-described objects, the following aspects of the invention are provided.

One aspect according to the present invention is a biological information measuring system comprising: a plurality of measuring devices which measure biological information of respective sites of a body; and a control device which is structured separately from the plurality of measuring devices, and which displays biological information obtained in measurement by the plurality of measuring devices with time, wherein the control device includes: start signal transmitting means for transmitting a measurement start signal generated in accordance with an input by a user operation to each of the plurality of measuring devices; measurement result receiving means for receiving biological information from each of the plurality of measuring devices; and displaying means for displaying the biological information obtained in the reception by the measurement result receiving means with time. On the other hand, each of the plurality of measuring devices includes: measurement start signal receiving means for receiving a measurement start signal from the control device; measuring means for starting measurement of biological information after receiving the measurement start signal by the measurement start signal receiving means; and measurement result transmitting means for transmitting measurement results obtained in measurement by the measuring means to the control device.

In accordance with the invention, it is possible to measure biological information at respective body sites simultaneously by a plurality of measuring devices in response to a measurement starting operation serving as a user operation with respect to one control device, and to display measurement results thereof at the one control device. Therefore, it is possible to certainly and easily confirm the relationship among the biological information of the plurality of body sites. As a result, for example, it is possible to certainly and easily confirm the relationship among the active states of a plurality of muscles during exercise.

Further, in accordance with the invention, because the measuring devices measuring biological information and the control device displaying the biological information with time are provided separately, it is possible for a user not only to carry out a measurement starting operation with respect to the control device at hand, but also to certainly recognize the measurement results visually in real time at the control device during measurement.

Further, another aspect according to the invention comprises: a plurality of measuring means for continuously measuring biological information of predetermined sites of a human body; displaying means for displaying measurement results obtained by measuring the biological information by the plurality of measuring means with time; and display control means for controlling a display on the displaying means such that the measurement results are displayed in parallel for the each measuring means.

In accordance with the invention, because measurement results obtained by measuring biological information by the plurality of measuring means are displayed in parallel for each of the measuring means on the displaying means, it is possible to achieve the effect that a user can carry out an appropriate exercise in consideration of a balance in the entire body and the like by comparison and contrast with situations in his/her body at a plurality of sites.

Yet another aspect according to the invention comprises: measuring means for continuously measuring biological information of a predetermined site of a human body; detecting means for detecting a point in time of activity start and a point in time of activity end of the predetermined site on the basis of measurement values measured by the measuring means; calculating means for calculating activity efficiency on the basis of an activity time from a point in time of activity start to a point in time of activity end detected by the detecting means, and an activity interval from the point in time of activity start up to a time when a following point in time of activity start is detected by the detecting means; and display control means for controlling a display of the activity efficiency calculated by the calculating means.

In accordance with the invention, it is possible to measure biological information of a predetermined site of a human body, and to calculate activity efficiency on the basis of an activity time and an activity interval at the predetermined site. Further, it is possible to show a user the activity efficiency to be displayed.

Yet another aspect according to the invention comprises: measuring means for measuring biological information of respective body portions; condition setting means for setting a measurement condition in the measurement of biological information by the measuring means; displaying means for displaying measurement results obtained in measurement of the biological information by the measuring means with time; and display cycle setting means for setting a cycle in which the measurement results are displayed on the displaying means in accordance with the measurement condition set by the condition setting means.

In accordance with the invention, it is possible to display changes over time in biological information such as a fatigue degree of a body such as a muscle state, a heart pulse rate, and a blood pressure according to an exercise or the like, in a graph, for example. In accordance therewith, it is possible to achieve the effect that a user can grasp a condition of his/her own body with time, and to carry out an exercise, a body movement, or the like suitable for the body condition. Further, when an exercise or the like is carried out, duration, the number of movements, and the like of the exercise differ depending on a type of the exercise or a person (the user) who carries out the exercise. In this point, in accordance with the present invention, since a cycle in which measurement results are displayed is set in accordance with a measurement condition in the measurement of biological information, it is possible to display a graph showing changes over time in the biological information, or the like, so as to be easily viewable in accordance with duration, the number of movements, and the like of the exercise.

The invention can be more fully understood from the following detailed description when taken in conjunction with the accompanying drawings, in which:

FIG. 1 is a diagram showing an example of use of a biological information measuring system of the present invention;
FIG. 2 is a block diagram showing examples of control structures of a myoelectric potential measuring device and a control device which form the biological information measuring system of the present invention;
FIG. 3A is a graph showing an example of a myoelectric waveform of a left leg; FIG. 3B is a graph showing an example of a myoelectric waveform of a right leg;
FIG. 4 is a diagram showing a structural example of a memory unit of the myoelectric potential measuring device;
FIG. 5 is a diagram showing a concrete structural example of the memory unit of the control device;
FIG. 6 is a diagram showing an example of a data structure of muscle state data;
FIG. 7A is a graph schematically showing data contents of pedaling efficiency data;
FIG. 7B is a graph schematically showing data contents of pedaling efficiency data;
FIG. 8A is a graph schematically showing data contents of average pedaling efficiency data;
FIG. 8B is a graph schematically showing data contents of average pedaling efficiency data;
FIG. 9 is a flowchart for explanation of flow of processes in the myoelectric potential measuring device and the control device;
FIG. 10 is a flowchart for explanation of flow of processes in the myoelectric potential measuring device and the control device;
FIG. 11A is a view showing one example of a graph displayed on a display unit in a short-term mode; FIG. 11B is a view showing one example of a graph displayed on the display unit in a medium-term mode; and
FIG. 11C is a view showing one example of a graph displayed on the display unit in a long-term mode.

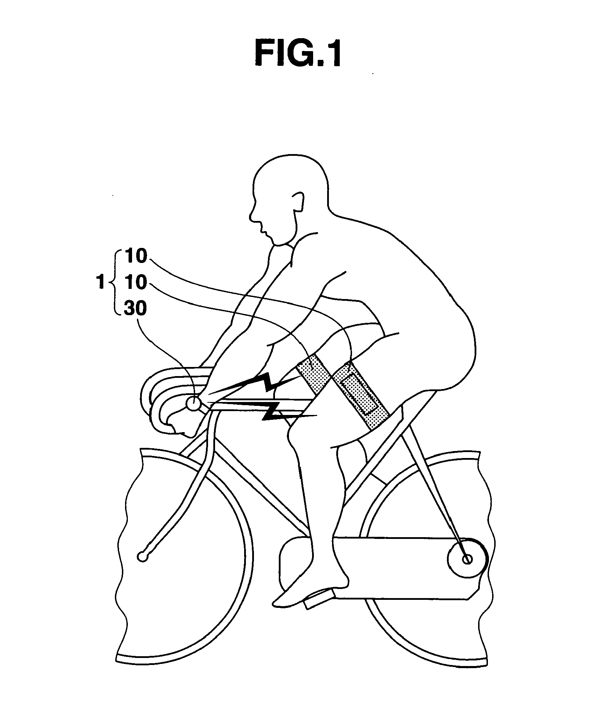

Hereinafter, preferred embodiments of a biological information measuring system of the present invention will be described in detail with reference to FIGS. 1 to 11C. Note that a case in which myoelectric potentials in a cycling exercise are measured as biological information will be described as an example hereinafter.

FIG. 1 is a diagram showing a state in which a user wears a biological information measuring system 1. The biological information measuring system 1 includes a plurality of myoelectric potential measuring devices 10 serving as measuring tools which can be mounted on respective body sites of the user, and a wristwatch control device 30 which is separated from the respective myoelectric potential measuring devices 10, and is capable of carrying out data communication with the respective myoelectric potential measuring devices 10. In the biological information measuring system 1 according to the embodiment, when myoelectric potentials in a cycling exercise are measured, for example, as shown in FIG. 1, the myoelectric potential measuring devices 10 are respectively mounted on the musculus vastus lateralis of femoral quadriceps of the both legs, and the control device 30 is mounted on an arm.

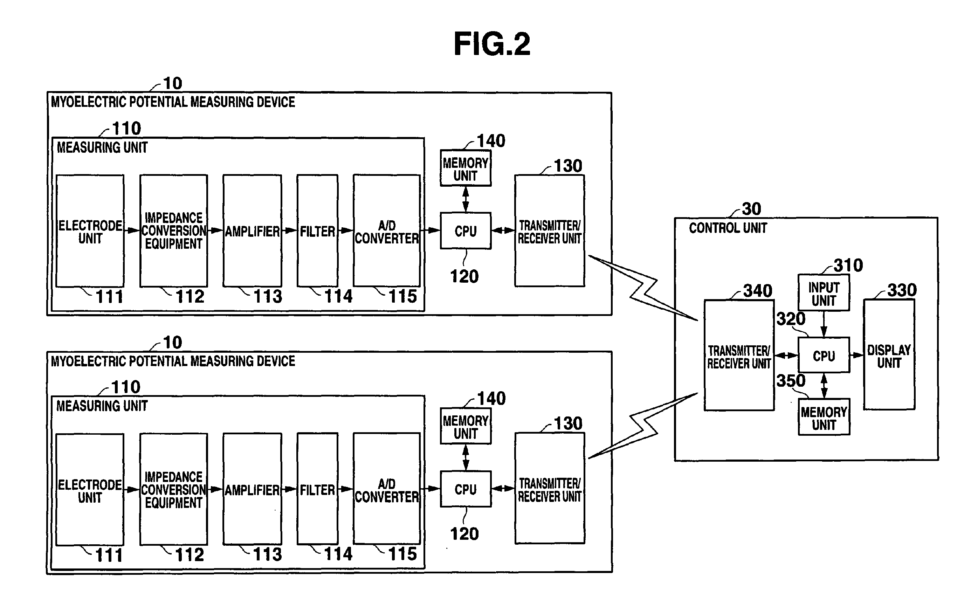

At the time of mounting those, the user sticks electrode units 111 (refer to FIG. 2) of the myoelectric potential measuring devices 10 respectively on the musculus vastus lateralis of femoral quadriceps of the both legs so as to be fixed by bands or the like, and wears the control device 30 on his/her wrist. Then, the user starts to measure myoelectric potentials by operating the control device 30, and starts an exercise by pedaling. Alternatively, it is possible for the user to start measuring myoelectric potentials in the middle of the exercise.

In the case where such a cycling exercise is carried out, the muscles of the both legs repeat an activity and a pause. Thus, provided that active states and resting states of the muscles are detected on the basis of myoelectric potentials, it is possible to obtain a state of changes over time in the muscular movement.

[Control Structure]

FIG. 2 is a block diagram showing examples of control structures of the myoelectric potential measuring devices 10 and the control device 30 which structure the biological information measuring system 1.

First, the control structure of the myoelectric potential measuring device 10 will be described. The myoelectric potential measuring device 10 is structured to have a measuring unit 110, a CPU 120, a transmitter/receiver unit 130, and a memory unit 140.

The measuring unit 110 among those is an operation part measuring a myoelectric potential, and is structured from: an electrode unit 111 which is formed from a pair of electrodes made to contact the skins of the respective body sites such as the musculus vastus lateralis described above, and which detects a potential difference between the pair of electrodes with time; an impedance converter 112 which converts the potential difference detected by the electrode unit 111 into a low impedance signal to be outputted; an amplifier 113 which amplifies the signal inputted from the impedance converter 112 to a predetermined signal level to be outputted; a filter 114 which eliminates frequency components out of range by making a signal within a predetermined frequency range among signals inputted from the amplifier 113 pass through; and an A/D converter 115 which analog-to-digital converts the signal inputted from the filter 114 to be outputted.

To describe concretely, a change in the electrical potential between two points detected by the electrode unit 111 is several tens $\mu$V to several mV, and a frequency band of the myoelectric potential signal is 2 KHz to 10 KHz. Since an impedance of a living body is generally extremely high, impedance conversion is carried out by the impedance converter (for example, a voltage follower type circuit) 112. Next, a voltage is amplified about hundredfold by the amplifier (for example, an operational amplifier) 113, which makes it possible to process a myoelectric potential waveform. Various noises are superimposed on a waveform amplified by the amplifier 113. Then, frequency components out of range processed as a myoelectric waveform are eliminated by the filter 114 at the subsequent stage. Next, a signal (an analog signal) inputted from the filter 114 is converted into a digital signal by the A/D converter (for example, a twelve-bit A/D converter) 115.

Note that, here, an analog filter has been used as the filter 114. In place of the analog filter, a digital filter may be used. In this case, the digital filter is provided at a subsequent stage of the A/D converter 115.

The CPU 120 controls the myoelectric potential measuring device 10 by issuing instructions to the respective operation parts in the myoelectric potential measuring device 10, carrying out data transfer, and the like on the basis of a program and data stored in the memory unit 140, data transmitted from the control device 30, and the like. Further, in order to achieve the present embodiment, the CPU 120 executes muscle state measurement process in which an active state of muscles (a muscle state) is measured on the basis of myoelectric potentials (biological information) continuously measured by the measuring unit 110, and the measurement result is transmitted to the control device 30.

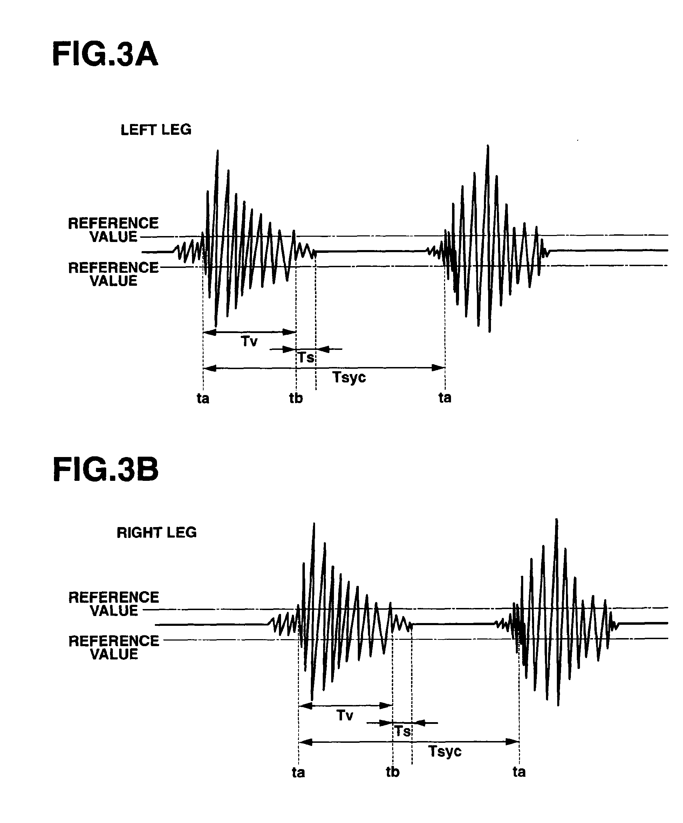

This muscle state measurement process will be described concretely with reference to FIGS. 3A and 3B. The drawing is a diagram showing examples of myoelectric potential waveforms. In the drawing, a waveform of myoelectric potential measured by the measuring unit 110 of the myoelectric potential measuring device 10 for left leg stuck to the musculus vastus lateralis on the left leg is shown in FIG. 3A, and a waveform of myoelectric potential measured by the measuring unit 110 of the myoelectric potential measuring device 10 for right leg stuck to the musculus vastus lateralis on the right leg is shown in FIG. 3B. Note that the actual muscle state measurement process is carried out by computing, by the CPU 120, a value of a signal digitized by the A/D converter 115. However, for convenience of explanation, here, the description will be given by using analog myoelectric waveforms of FIGS. 3A and 3B.

In this muscle state measurement process, the CPU 120 detects a muscle activity start timing (ta) serving as a point in time of muscle activity start, and a muscle activity end timing (tb) serving as a point in time of muscle activity end based on the measured myoelectric potential, and carries out measurement of a muscle state for each pedaling.

To describe concretely, the CPU 120 judges that a point in time when a value of a measured myoelectric potential is made greater than or equal to a reference value (a predetermined threshold value) (hereinafter referred to as "timing") as a muscle activity start timing (ta). Further, the CPU 120 stores changes over time in myoelectric potential from the muscle activity start timing (ta) as myoelectric potential generating data which will be described later, into a myoelectric generating data area 142 of the memory unit 140. Then, the CPU 120 monitors whether or not a value of the myoelectric potential is made less than the reference value. When the value of the myoelectric potential is made less than the reference value, the CPU 120 judges whether or not a value of myoelectric potential measured from that point in time to a predetermined time (Ts) is still less than the reference value. When the value of the myoelectric potential is still less than the reference value, a point in time when the value is made less than the reference value described above is defined as a muscle activity end timing (tb). Further, the CPU 120 calculates a time from a muscle activity start timing (ta) up to a muscle activity end timing (tb), i.e., a time for which the muscles are in activity in the pedaling (of pedaling), as muscular strength keeping duration (Tv), and stores it into the memory unit 140.

Note that, here, a timing when a value of the measured myoelectric potential is made greater than or equal to the reference value has been judged as a muscle activity start timing (ta). However, when the number of times a value of the measured myoelectric potential is made greater than or equal to the reference value within a predetermined time reaches a predetermined number of times, a point in time when the value is made greater than or equal to the reference value for the first time within the predetermined time may be judged as a muscle activity start timing (ta). Alternatively, when the number of times a value of the measured myoelectric potential is made greater than or equal to the reference value within a predetermined time reaches a predetermined number of times, a point in time when the value is made greater than or equal to the reference value for the last time within the predetermined time may be judged as a muscle activity start timing (ta). The same applies to the following. This is for preventing an error caused by a noise included in a waveform of myoelectric potential at the start of measurement, and for preventing the myoelectric potential measuring device 10 from erroneously operating by the noise.

Thereafter, when the value of the measured myoelectric potential is made greater than or equal to the reference value again, the CPU 120 judges that the next muscle activity is started at that point in time. In this case, the CPU 120 calculates a time from the previous muscle activity start timing (ta) up to the point in time, namely, a time from the previous pedaling up to the next pedaling, i.e., a time which has been required for one pedaling, as a myoelectric potential generating interval (Tsyc) serving as an activity interval, and updates the number of pedaling serving as the number of activity occurrences. Then, the CPU 120 calculates an averaged rectified value (ARV) serving as an index for evaluating muscle tensile force on the basis of myoelectric generating data serving as a change in the myoelectric potential in the previous pedaling, and a mean power frequency (MPF) serving as an index for evaluating muscle fatigue, or the like.

Namely, the CPU 120 obtains the averaged rectified value (ARV) by processing an amplitude value on the basis of the obtained myoelectric waveform. This averaged rectified value is an index showing a performed muscular strength. Further, the CPU 120 determines a mean power frequency (MPF) or the like by FFT-measuring the obtained myoelectric waveform. This mean power frequency is an index showing a muscle fatigue degree.

Note that, in this example, the CPU 120 is configured to calculate an averaged rectified value (ARV), a mean power frequency (MPF) and the like. However, indices calculated by the CPU 120 are not limited to these values. For example, on the basis of the myoelectric potential generating data described above, a root-mean-square-value (RMS) and an integral value mainly relating to muscle tensile force, or a median frequency relating to muscle fatigue may be calculated.

Then, the muscle activity start timing (ta) is updated, and the myoelectric potential generating data 142 is reset, thereby measuring a muscle state relating to the following pedaling by carrying out the above-described processing.

The transmitter/receiver unit 130 is an operation part for carrying out wireless communication with the control device 30, and demodulates a signal transmitted from the control device 30 via, for example, an antenna (not shown), and outputs the signal to the CPU 120. On the other hand, the transmitter/receiver unit 130 modulates and amplifies a control signal from the CPU 120, and transmits the signal to the control device 30 via the antenna.

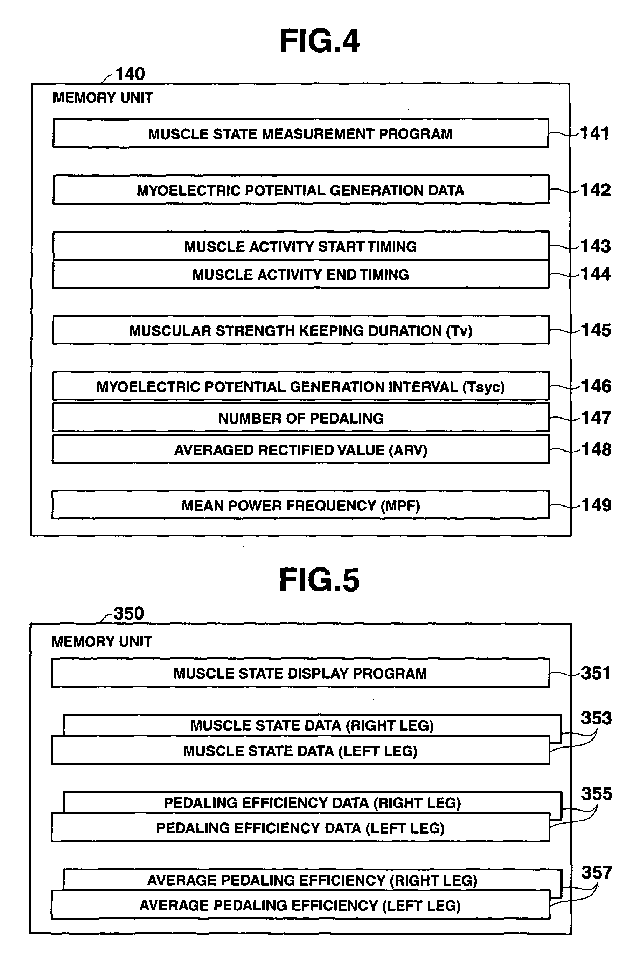

The memory unit 140 is realized by an IC memory such as a ROM and a RAM, an information storage medium such as a hard disk, and a reading device thereof, and a program for causing the CPU 120 to control the myoelectric potential measuring device 10, and the like are stored therein. FIG. 4 is a diagram showing a concrete structural example of the memory unit 140, and in order to realize the present invention, a muscle state measurement program for executing the muscle state measurement process described above is stored in a muscle state measurement program area 141. Further, as data, myoelectric potential generating data, a muscle activity start timing (ta), a muscle activity end timing (tb), muscular strength keeping duration (Tv), a myoelectric potential generating interval (Tsyc), the number of pedaling, an averaged rectified value (ARV), and a mean power frequency (MPF) are respectively stored in a myoelectric potential generating data area 142, a muscle activity start timing (ta) area 143, a muscle activity end timing (tb) area 144, muscular strength keeping duration (Tv) area 145, a myoelectric potential generating interval (Tsyc) area 146, a number-of-pedaling area 147, an averaged rectified value (ARV) area 148, and a mean power frequency (MPF) area 149, and those are respectively rewritten to be updated for each cycle of pedaling.

Next, the control structure of the control device 30 will be described. As shown in FIG. 2, the control device 30 is structured to have an input unit 310, a CPU 320, a display unit 330, a transmitter/receiver unit 340, and a memory unit 350.

The input unit 310 among those is realized by, for example, various switches and dials, touch panels, and the like, and outputs an operation signal according to an operational input to the CPU 320. Inputs of a measurement start instruction and a measurement end instruction of myoelectric potential are carried out by the function of the input unit 310. Further, the input unit 310 functions as condition setting means for setting a measurement condition in the measurement of biologic information by the myoelectric potential measuring device 10. In the present embodiment, a case in which an exercise mode is set as a measurement condition will be described as an example. The myoelectric potential measuring device 10 is to carry out measurement of a myoelectric potential in accordance with an exercise mode inputted by the input unit 310.

Here, setting of an exercise mode will be described. As an exercise mode set by the input unit 310, for example, there are three modes of a short term, a medium-term, and a long-term. The short-term mode is expected for an exercise at full power, for example, within 1 to 2 minutes. When the short-term mode is set as a measurement condition, for example, measurement by the myoelectric potential measuring device 10 is continuously carried out from the start of the measurement. Further, the medium-term mode is expected for an exercise for, for example, about 10 minutes. When the medium-term mode is set as a measurement condition, for example, measurement by the myoelectric potential measuring device 10 is repeated for a short time from the start of the measurement. Further, the long-term mode is expected for an exercise for over one hour such as a marathon. When the long-term mode is set as a measurement condition, for example, the myoelectric potential measuring device 10 repeatedly carries out measurement every five minutes.

The CPU 320 controls the control device 30 and the entire biological information measuring system 1 by issuing instructions to the respective operation parts in the control device 30, carrying out transfer of data, and the like on the basis of a program and data stored in the memory unit 350, an operation signal inputted from the input unit 310, data transmitted from the myoelectric potential measuring device 10, and the like. Further, in order to achieve the present embodiment, the CPU 320 executes muscle state measurement process in which a measurement start signal is transmitted to each of the myoelectric potential measuring devices 10 when a measurement start instruction is inputted from the input unit 310, and on the other hand, a display on the display unit 330 is controlled on the basis of a measurement result transmitted as needed from each of the myoelectric potential measuring devices 10.

In the present embodiment, when a measurement result of myoelectric potential measured in accordance with an exercise mode is transmitted from the myoelectric potential measuring device 10, the CPU 320 functions as display cycle setting means for setting a cycle in which the measurement result is displayed on the display unit 330, in accordance with an exercise mode serving as a measuring condition. The CPU 320 sets a cycle in which the measurement result is displayed on the display unit 330 in accordance with an exercise mode, and displays a graph or the line on the display unit 330 at the set cycle.

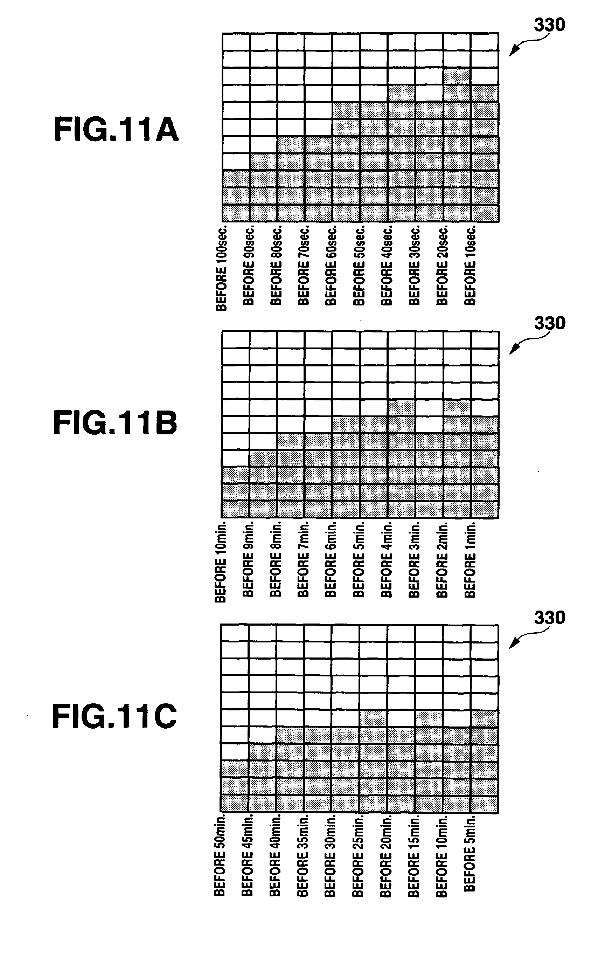

For example, when the short-term mode is inputted as an exercise mode, as shown in FIG. 11A, a measurement result of myoelectric potential, an active state of muscles, or the like is displayed at a cycle of ten seconds. In this case, the abscissa of the bar graph is set such that a measurement result of myoelectric potential measured for ten seconds, or an active state of muscles obtained by measuring the measurement result is made to be one bar in the bar graph. Note that, when the myoelectric potential measuring device 10 measures a myoelectric potential once per ten seconds, a measurement result of the myoelectric potential, or an active state of muscles is made to be one bar in the bar graph. Further, when the myoelectric potential measuring device 10 measures myoelectric potential several times per ten seconds, an average value of measurement results of the myoelectric potentials, or active states of muscles obtained by measuring the measurement results is made to be one bar in the bar graph.

In the same way, for example, when the medium-term mode is inputted as an exercise mode, as shown in FIG. 11B, a measurement result of myoelectric potential, an active state of muscles, or the like is displayed at a cycle of one minute. In this case, the abscissa of the bar graph is set such that a measurement result of myoelectric potential or an active state of muscles measured for one minute is made to be one bar in a bar graph. Note that, as in the short-term mode, when the myoelectric potential measuring device 10 measures a myoelectric potential once per minute, a measurement result of the myoelectric potential, or an active state of muscles is made to be one bar in the bar graph, and when the myoelectric potential measuring device 10 measures myoelectric potentials several times per minute, an average value of measurement results of the myoelectric potentials, or active states of muscles is made to be one bar in the bar graph.

Further, for example, when the long-term mode is inputted as an exercise mode, as shown in FIG. 11C, a measurement result of myoelectric potential, an active state of muscles, or the like is displayed at a cycle of five minutes. In this case, the abscissa of the bar graph is set such that a measurement result of myoelectric potential or an active state of muscles measured for five minutes is made to be one bar in a bar graph. Note that, when the myoelectric potential measuring device 10 measures a myoelectric potential once per five minutes and measures myoelectric potentials several times per five minutes, the same values as in the short-term mode and the medium-term mode are respectively used as one bar in the bar graph.

The display unit 330 is realized by a display device such as an LCD and an ELD, and displays a measurement result of myoelectric potential measured by the myoelectric potential measuring device 10, for example, an active state of measured muscles (biological information) in accordance with an instruction from the CPU 320. In the present embodiment, a width of the abscissa of the graph is set such that a graph is displayed on the display unit 330 at a display cycle set by the CPU 320 serving as display cycle setting means as described above, and the display unit 330 displays a graph showing a measurement result of myoelectric potential, an active state of muscles, and the like at the set display cycle.

The transmitter/receiver unit 340 is an operation part for carrying out wireless communication with the myoelectric potential measuring device 10, demodulates a signal transmitted from the myoelectric potential measuring device 10 via, for example, an antenna (not shown), and outputs the signal to the CPU 320, and modulates and amplifies a control signal inputted from the CPU 320, and outputs the signal to the measuring devices 10 via the antenna.

The memory unit 350 is realized by an IC memory such as a ROM and a RAM, an information memory unit such as a hard disk, and a read device thereof, and a program for making the CPU 320 control the control device 30 is stored therein. FIG. 5 is a diagram showing a concrete structural example of the memory unit 350, and in order to achieve the present embodiment, a muscle state display program 351 for executing muscle state display process described above is stored therein. Further, as data, muscle state data, pedaling efficiency data, and average pedaling efficiency data are respectively stored in a muscle state data area 353, a pedaling efficiency data area 355, and average pedaling efficiency data area 357.

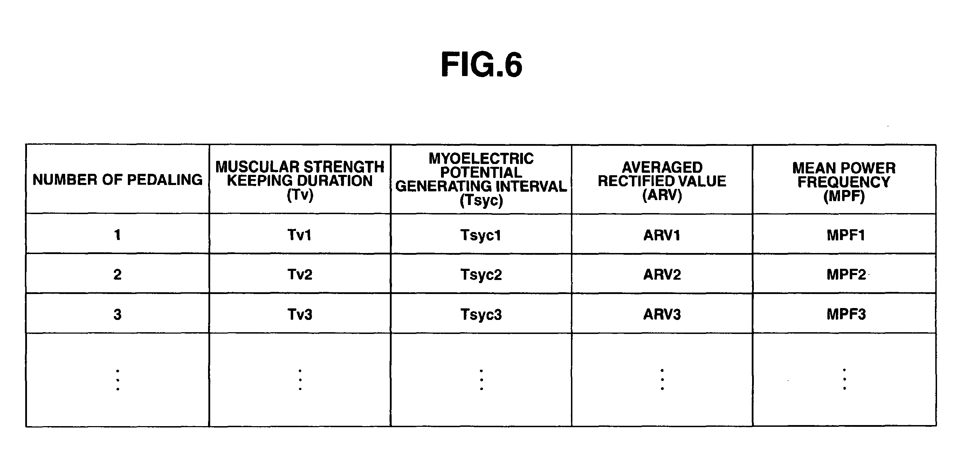

The muscle state data area 353 is prepared for each of the myoelectric potential measuring devices 10, and stores a measurement result transmitted from the corresponding myoelectric potential measuring device 10. FIG. 6 is an example of a data structure in the muscle state data area 353. As shown in the drawing, in the muscle state data area 353, respective values of the number of pedaling, muscular strength keeping duration (Tv), myoelectric potential generating intervals (Tsyc), averaged rectified values (ARV), and mean power frequencies (MPF) serving as measurement results are stored so as to correspond to the number of pedaling allocated in the order of receiving.

The pedaling efficiency data area 355 is prepared for each of the myoelectric potential measuring devices 10, and stores pedaling efficiency serving as an activity efficiency calculated on the basis of measurement results transmitted from the corresponding myoelectric potential measuring device 10. Hereinafter, a calculation formula (1) of the pedaling efficiency is shown.

$$\text{Pedaling efficiency} = (\text{muscular strength keeping duration (Tv)}/\text{myoelectric potential generating interval (Tsyc)}) \times 100 \qquad \dots(1)$$

Namely, the pedaling efficiency is a value showing a ratio that a time for which muscles are actually in activity accounts for in a single pedaling time, and the less the value is, the higher the pedaling efficiency is. Note that an inverse number thereof may be pedaling efficiency. In this case, the greater the value is, the higher the pedaling efficiency is.

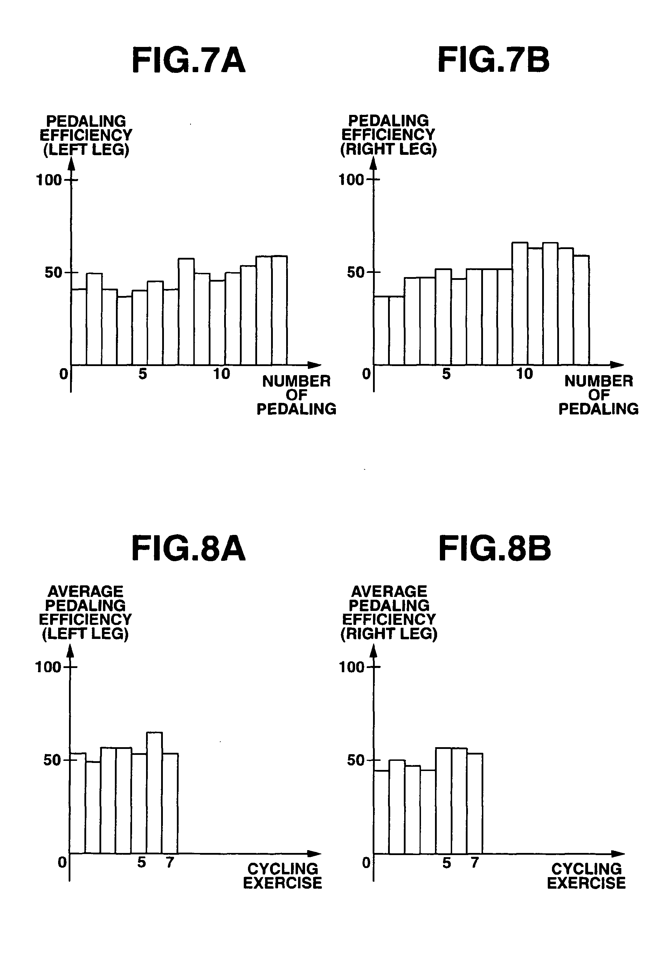

FIGS. 7A and 7B are graphs schematically showing data contends in the pedaling efficiency data area 355. In the graphs, the pedaling efficiency data based on the measurement results by the myoelectric potential measuring device 10 for left leg are shown in FIG. 7A, and the pedaling efficiency data based on the measurement results by the myoelectric potential measuring device 10 for right leg are shown in FIG. 7B. As shown in FIGS. 7A and 7B, the pedaling efficiency calculated by the calculation formula (1) described above are stored so as to correspond to the number of pedaling in the pedaling efficiency data area 355.

The average pedaling efficiency data area 357 is prepared for each of the myoelectric potential measuring devices 10, and stores an average value of pedaling efficiency obtained by a single cycling exercise. FIGS. 8A and 8B are graphs schematically showing data contents in the average pedaling efficiency data area 357. In the graphs, the average pedaling efficiency data based on the pedaling efficiency data for left leg are shown in FIG. 8A, and the pedaling efficiency data based on the pedaling efficiency data for right leg are shown in FIG. 8B. In the average pedaling efficiency data area 357, average pedaling efficiency at this point in time relating to a current cycling exercise is updated as needed on the basis of pedaling efficiency data, and is stored, and average pedaling efficiency finally obtained in the past cycling exercise is stored. Namely, for example, when cycling exercise currently is being carried out, average pedaling efficiency relating to the cycling exercise (the seventh cycling exercise in FIGS. 8A and 8B) is updated for each pedaling.

[Flow of Processes]

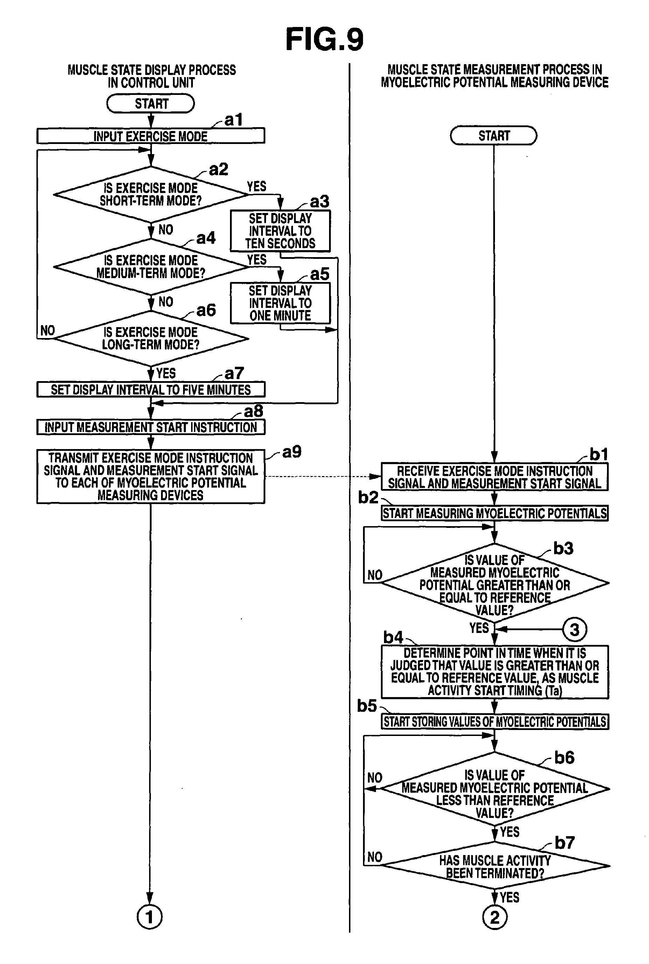

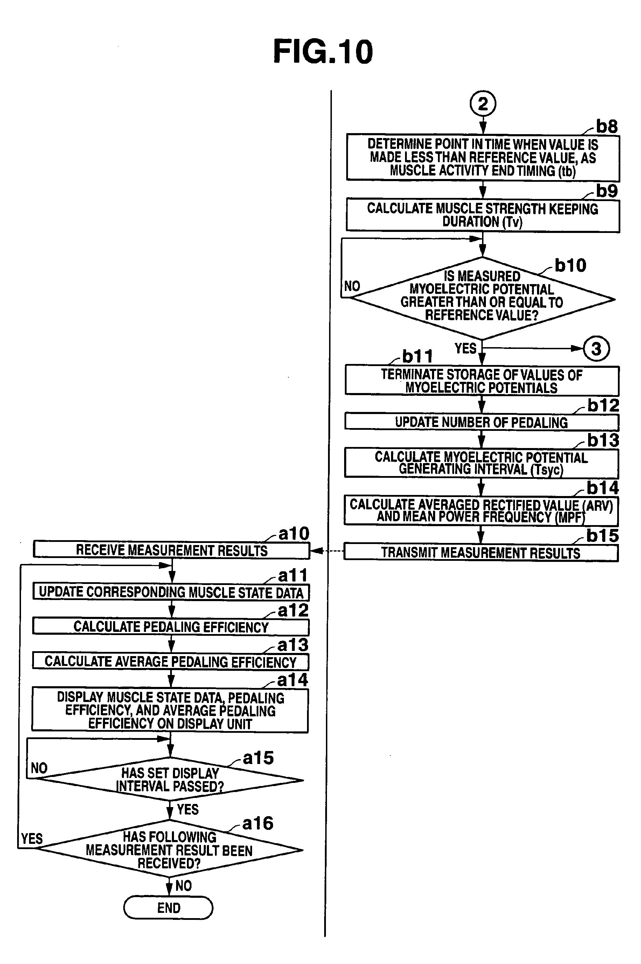

Next, flow of processes in the myoelectric potential measuring device 10 and the control device 30 will be described with reference to FIGS. 9 and 10. Note that the processes described here are processes which are achieved such that the CPU 120 of the myoelectric potential measuring device 10 reads a muscle state measurement program from the muscle state measurement program area 141 to execute the program, and the CPU 320 of the control device 30 reads a muscle state display program from the muscle state display program area 351 to execute the program. When a measurement end instruction is inputted from the input unit 310, or when a predetermined time passes on the basis of the program, the process is terminated in the control device 30.

Namely, first, when an exercise mode is inputted from the input unit 310 in the control device 30 (step a1), the CPU 320 judges whether or not the inputted exercise mode is a short-term mode (step a2). When it is the short-term mode (step a2: YES), a display cycle is set to ten seconds (step a3). When it is not the short-term mode (step a2: NO), the CPU 320 judges whether or not the inputted exercise mode is a medium-term mode (step a4). When it is the medium-term mode (step a4: YES), a display cycle is set to one minute (step a5). When it is not the medium-term mode (step a4: NO), the CPU 320 further judges whether or not the inputted exercise mode is a long-term mode (step a6). When it is the long-term mode (step a6: YES), a display cycle is set to five minutes (step a7). Note that, when it is judged that the inputted exercise mode is not the long-term mode (step a6: NO), the routine returns to step a2 again, and the judgment for an exercise mode is repeated.

Moreover, when a measurement start instruction is inputted from the input unit 310 in the control device 30 (step a8), the CPU 320 transmits an exercise mode instruction signal for instructing each of the respective myoelectric potential measuring devices 10 on a set exercise mode, and a measurement start signal for instructing on a measurement start (step a9).

In response thereto, in the myoelectric potential measuring device 10, the CPU 120 executes the muscle state measurement process. Namely, when the CPU 120 receives an exercise mode instruction signal and a measurement start signal (step b1), the CPU 120 starts measurement of myoelectric potentials by the measuring unit 110 at a measurement cycle corresponding to the instructed exercise mode (step b2). Note that the myoelectric potential measuring device 10 may always carry out measurement of myoelectric potential continuously regardless of an exercise mode. Hereinafter, a case in which the myoelectric potential measuring device 10 carries out measurement of myoelectric potential continuously regardless of a set exercise mode will be described.

The CPU 120 compares values of the myoelectric potential continuously measured by the measuring unit 110 with a reference value. When a value of measured myoelectric potential (for example, an amplitude) is made greater than or equal to the reference value (step b3: YES), the CPU 120 judges that point in time as a muscle activity start timing (ta) area 143 (step b4).

In this case, the CPU 120 starts to record the myoelectric potential from the determined muscle activity start timing (ta), and stores it in the myoelectric generating data area 142 of the memory unit 140 (step b5). Further, the CPU 120 monitors changes in the myoelectric potential to be measured, and when a value of the myoelectric potential (for example, an amplitude) is made less than the reference value (step b6: YES), as described above with reference to FIG. 3, the CPU 120 judges whether or not a value of the myoelectric potential measured from that point in time up to a time when a predetermined time (Ts) has passed is still less than the reference value. Then, the CPU 120 judges that the muscle activity is completed when the value is still less than the reference value (step b7: YES), and stores the point in time when the value is made less than the reference value judged at step b6 into the muscle activity end timing (tb) area 144 (step b8). At the same time, the CPU 120 calculates, as muscular strength keeping duration (Tv), a time from the muscle activity start timing (ta) stored in the muscle activity start timing (ta) area 143 to the muscle activity end timing (tb) stored in the muscle activity end timing (tb) area 144, (step b9), and stores the time into the muscular strength keeping duration (Tv) area 145 of the memory unit 140.

Then, when the value of a myoelectric potential measured by the measuring unit 110 is made greater than or equal to the reference value (step b10: YES), the CPU 120 returns to step b4 in order to obtain the myoelectric potential of a following pedaling, judges a point in time when a value is made greater than or equal to the reference value as the next muscle activity start timing (ta), and carries out measurement of the next pedaling through the steps from step b4 to step b10.

On the other hand, at a point in time when a value of the myoelectric potential measured by the measuring unit 110 at step b10 is made greater than or equal to the reference value (step b10: YES), the CPU 120 terminates storage of the previous myoelectric potential (step b11). Then, the CPU 120 updates the number of pedaling (step b12), calculates a time from the muscle activity start timing (ta) to the muscle activity end timing (tb) determined at step b4, as a myoelectric potential generating interval (Tsyc) (step b13), and stores the calculated result into the myoelectric potential generating interval (Tsyc) area 146. Next, the CPU 120 calculates an averaged rectified value (ARV) and a mean power frequency (MPF) on the basis of the obtained myoelectric potential generating data of the last pedaling (step b14), and stores those respectively into the averaged rectified value (ARV) area 148 and the mean power frequency (MPF) area 149 of the memory unit 140.

Next, the CPU 120 transmits the respective values of the muscular strength keeping duration (Tv) 145 calculated at step b9, the myoelectric potential generating interval (Tsyc) 146 calculated at step b13, and the averaged rectified value (ARV) 148 and the mean power frequency (MPF) 149 calculated at step b14 so as to correspond to the number of pedaling updated at step b12, as measurement results to the control unit 30 (step b15) .

On the other hand, in the control unit 30, when the CPU 320 receives the measurement results (step a10), the CPU 320 stores the received measurement results additionally into the muscle state data area 353 for the myoelectric potential measuring device 10 which has transmitted the measurement results (step a11). Namely, the measurement results received from the myoelectric potential measuring device 10 for left leg are additionally stored into the muscle state data area 353 for left leg, and the measurement results received from the myoelectric potential measuring device 10 for right leg are additionally stored into the muscle state data area 353 for right leg.

Next, the CPU 320 calculates pedaling efficiency (step a12). To describe concretely, the CPU 320 reads out respective values of the muscular strength keeping duration (Tv) and the myoelectric potential generating interval (Tsyc) from the measurement results added to the muscle state data area 353 at step a40. Then, the CPU 320 calculates pedaling efficiency in accordance with the calculation formula (1) described above, and carries out process for storing calculated pedaling efficiency data, so as to correspond to the numbers of pedaling, additionally into the pedaling efficiency data area 355, for each of the left leg and the right leg.

Next, the CPU 320 calculates average pedaling efficiency (step a13). Namely, the CPU 320 calculates an average value of the pedaling efficiency stored in the pedaling efficiency data area 355 as average pedaling efficiency relating to the latest cycling exercise, and carries out process of updating average pedaling efficiency data stored in average pedaling efficiency data area 357, for each of the left leg and the right leg.

Then, the CPU 320 controls a display on the display unit 330 on the basis of the respective data of the muscle state data, the pedaling efficiency data, and the average pedaling efficiency data (step a14).

For example, the CPU 320 sets the abscissa of the graph to be displayed on the display unit 330 so as to be a display cycle corresponding to a set exercise mode, and displays changes over time in the respective values of the muscular strength keeping duration (Tv), the myoelectric potential generating interval (Tsyc), the averaged rectified value (ARV), and the mean power frequency (MPF), the pedaling efficiency, the average pedaling efficiency relating to the latest cycling exercise in a graph on the display unit 330 so as to be contrasted with one another with respect to the left leg and the right leg. Further, the average pedaling efficiency relating to the past cycling exercise may be displayed in a graph in addition to the average pedaling efficiency relating to the latest cycling exercise, and for example, those are displayed in a graph so as to be contrasted with one another with respect to the left leg and the right leg.

In accordance therewith, it is possible to measure information on tensions and muscle fatigues in the both legs measured on the basis of the myoelectric potential for each pedaling, and to show it to the user. Further, since it is possible to calculate the respective values of the pedaling efficiency and the average pedaling efficiency for each pedaling to be shown to the user, the user can exercise with reference to these values as pace allocation.

Moreover, provided that the graph is displayed such that the values are contrasted with each other with respect to the left leg and the right leg, it is possible for the user to easily grasp the left-right symmetry (the balance or the like).

Moreover, the CPU 320 always judges whether or not a timing of the next display cycle corresponding to a set exercise mode has passed (step a15), and unless a timing of the next display cycle has passed (step a15: NO), the CPU 320 repeats a judgment at step a15 while displaying the measurement results on the display unit 330. On the other hand, when the display cycle has passed (step a15: YES), the CPU 320 judges whether or not the next measurement results have been received (step a16), and when the next measurement results have been received (step a16: YES), the CPU 320 repeats the processes from step a11 to step a14, and displays the newly obtained measurement results so as to be next to the previously displayed measurement results on the display unit 330. On the other hand, when the next measurement results have not been received (step a16: NO), the CPU 320 terminates the muscle state display process.

As described above, in accordance with the present embodiment, it is possible to measure muscle states of the corresponding sites on the basis of measured myoelectric potential by the myoelectric potential measuring devices 10 mounted on respective body sites, and to update and display those as needed in the control device 30. Further, at this time, since they are displayed at a display cycle corresponding to an exercise mode set in advance, in both of a short-term mode in which an exercise time is short and a long-term mode in which an exercise time is long, they can be displayed in a display format easily viewable for a user. Moreover, because measurement results of myoelectric potential measured at the respective sites can be displayed so as to be contrasted with one another, it is possible for a user to exercise while comparing differences in the muscle states of the respective sites.

Note that, in the embodiment described above, the case in which myoelectric potential during a cycling exercise is measured has been described as an example. However, in the same way, the present invention can be applied to a case in which myoelectric potential during another exercise such as walking, jogging, or a marathon is measured.

Further, in the embodiment described above, the case in which the myoelectric potential measuring devices 10 are mounted on the two places of the musculus vastus lateralis of the both legs and myoelectric potentials thereof are measured, has been described. However, places onto which the myoelectric potential measuring devices 10 are mounted are not limited to this case, and it goes without saying that the myoelectric potential measuring devices 10 may be mounted on other muscles in which to measure muscle states by measuring myoelectric potentials, for example, other leg muscles such as musculus rectus femoris, musculus biceps femoris, or musculus soleus, and arm muscles such as musculus biceps brachii and musculus triceps brachii. Further, places onto which the myoelectric potential measuring devices 10 are mounted are not limited to two places, and those may be mounted on three or more places, or may be mounted on only one place.

Further, the embodiment described above functions as condition setting means for inputting an exercise mode (a measurement condition) from the input unit 310 of the control device 30 by the user, and it is configured such that the CPU 320 sets a display cycle by inputting an exercise mode from the input unit 310. However, the condition setting means is not limited to the one illustrated by an example. For example, it may be configured such that the CPU 320 automatically sets an exercise mode on the basis of the number of pedaling per minute (cadence), and determines a display cycle. In this case, the CPU 320 functions as condition setting means for setting a measurement condition.

For example, when the number of pedaling per minute is large, the CPU 320 judges that an exercise mode (a measurement condition) is a short-term mode, and sets a display cycle to be short, and when the number of pedaling per minute is small, the CPU 320 judges that an exercise mode (a measurement condition) is a long-term mode, and sets a display cycle to be long. In this case, a table is stored in the memory unit 350 of the control device 30. In the table, which exercise mode is to be set is associated with the number of pedaling per minute (cadence), and the CPU 320 sets an exercise mode (a measurement condition) with reference to the table. Note that threshold values may be set in advance, or may be set arbitrarily by a user. The short-term mode or the long-term mode is defined depending on whether or not the number of pedaling per minute (cadence) is greater than or equal to a predetermined one of the threshold values.

Further, in the embodiment described above, as shown in FIGS. 11A to 11C, time has been set as the abscissa of the graph displayed on the display unit 330. However, a display cycle of the graph may be changed such that the number of pedaling is set as the abscissa, and for example, the graph is displayed for each pedaling in a short-term mode, and the graph is displayed every tenth pedaling in a long-term mode.

Further, in the embodiment described above, a display cycle at the time of displaying on the display unit 330 is constant in each exercise mode. However, a display cycle may be changed in the middle of the display. For example, in a case in which a display is carried out every minute, when an exercise time duration exceeds over thirty minutes, an average value of results measured up to the time is made in a lot to be one graph, and a display may be carried out in a display cycle of a minute from the following one minute. Further, when an exercise for one day is completed, an average value of measurement results of the day is made in a lot to be one graph at that point in time, and at the time of exercising next time, a new measurement result may be displayed in accordance with the set display cycle next to the graph of the average value in the previous exercise.

Moreover, the control device 30 may be structured as follows. Namely, it may be structured such that respective data of muscle state data, pedaling efficiency data, and average pedaling efficiency data which is stored in the memory unit 350 are stored in a portable information storage medium such as a memory card so as to be utilizable at an external device such as a personal computer. Alternatively, an operation part for transmitting the respective data described above may be provided.

Further, in order to realize the transmitter/receiver units 130 and 340 inexpensively, it may be structured such that the above-described exercise modes can be set by the respective myoelectric potential measuring devices 10, and the respective myoelectric potential measuring devices 10 have only a transmitter function of the transmitter/receiver functions, and on the other hand, the control device 30 includes only a receiver function of the transmitter/receiver functions. In this case, an input unit is required in the myoelectric potential measuring device 10.

Moreover, in order to easily compare a current displayed measurement result with the previous measurement result, for example, in the control device 30, the current measurement result may be displayed such that a color is changed in accordance with an increase or a decrease in the following pitch with respect to the previous pitch (cycle). For example, when the pitch is increased, the color is "red", and when the pitch is decreased, the color is "green", and when there is no change in the pitch, the color is "yellow".

Further, means for inputting an intention to increase or decrease with respect to a current pitch (cycle) is provided, and a pitch gradually increased or decreased on the basis of the current pitch may be displayed in the control device 30. Alternatively, in addition thereto, a pitch may be displayed so as to increase or decrease the pitch timing in a designated direction in accordance with the state of changes in detected pitch.

Further, in the present embodiment, the system in which the myoelectric potential measuring devices 10 and the control device 30 are respectively provided so as to be separated from one another has been described. However, this may be a biological information measuring device in which measuring means for measuring myoelectric potentials, condition setting means for setting a measurement condition in the measurement of biological information by the measuring means, displaying means for displaying measurement results obtained by measuring the biological information by the measuring means with time, and display cycle setting means for setting a cycle in which the measurement results are displayed on the displaying means in accordance with the measurement condition set by the condition setting means, are provided in one device.

Further, in the present embodiment, the case in which myoelectric potentials serving as biological information are measured by the myoelectric potential measuring devices 10 has been described. However, objects to be measured by the measurement devices (measuring means) are not limited to myoelectric potentials. For example, those may broadly include biological information of a human body such as a blood pressure, a heart rate, and a pulse.

**Claims**

1. A biological information measuring device **characterized by** comprising:

   measuring means (10) for continuously measuring biological information of a predetermined site of a human body;
   detecting means (320) for detecting a point in time of activity start and a point in time of activity end of the predetermined site on the basis of measurement values measured by the measuring means (10) ;
   calculating means (320) for calculating activity efficiency on the basis of an activity time from a point in time of activity start up to a point in time of activity end detected by the detecting means (320), and an activity interval from the point in time of activity start up to a time when a next point in time of activity start is detected by the detecting means (320); and
   display control means (320) for controlling a display of the activity efficiency calculated by the calculating means (320).

2. The biological information measuring device according to claim 1, **characterized in that** the detecting means (320) includes comparison means (b3, b6) for comparing measurement values measured by the measuring means with a predetermined threshold value, detects a point in time when a measurement value exceeds the predetermined threshold value, as a point in time of activity start, and detects a point in time when a measurement value is made less than the predetermined threshold value after the point in time of activity start, as a point in time of activity end.

3. The biological information measuring device according to claim 1, **characterized by** further comprising

   counting means (320) for counting a number of activity occurrences at an activity interval,
   wherein the display control means (320) controls a display of the activity efficiency calculated by the calculating means (320) and the number of activity occurrences counted by the counting means (320).

4. The biological information measuring device according to claim 1, **characterized in that** the display control means (320) includes change display control means for displaying changes over time in the activity efficiency calculated by the calculating means (320).

5. The biological information measuring device according to claim 1, **characterized in that** the display control means (320) includes average value display control means for controlling a calculation and a display of an average value of the activity efficiency calculated by the calculating means (320).

6. A biological information measuring device **characterized by** comprising:

   a plurality of measuring means (10) for continuously measuring biological information of predetermined sites of a human body;
   displaying means (330) for displaying measurement results obtained by measuring the biological information by the plurality of measuring means (10) with time; and
   display control means (320) for controlling a display on the displaying means (330) such that the measurement results are displayed in parallel for the each measuring means (10).

7. The biological information measuring device according to claim 6, **characterized by** further comprising:

detecting means (320) for detecting a point in time of activity start and a point in time of activity end of the predetermined sites on the basis of measurement values measured by the plurality of measuring means (10); and calculating means (320) for calculating activity efficiency on the basis of an activity time from a point in time of activity start up to a point in time of activity end detected by the detecting means (320), and an activity interval from the point in time of activity start up to a time when a next point in time of activity start is detected by the detecting means (320),
wherein the display control means (320) controls to display the activity efficiency calculated by the calculating means (320) in parallel for the each measuring means (10) in the displaying means (330).

8. The biological information measuring device according to claim 6, **characterized in that** the detecting means (320) includes comparison means (b3, b6) for comparing measurement values measured by the measuring means (10) with a predetermined threshold value, detects a point in time when a measurement value exceeds the predetermined threshold value, as a point in time of activity start, and detects a point in time when a measurement value is made less than the predetermined threshold value after the point in time of activity start, as a point in time of activity end.

9. A biological information measuring system **characterized by** comprising:

a plurality of measuring devices (10) which continuously measure biological information of predetermined sites of a human body; and
a control device (30) which displays measurement results obtained by measuring the biological information by the plurality of measuring devices (10) with time,
wherein the control device (30) includes:

displaying means (330) for displaying the measurement results obtained by measuring the biological information by the plurality of measuring devices (10) with time; and
display control means (320) for controlling a display on the displaying means (330) such that the measurement results are displayed in parallel for the each measuring device (10).

10. A biological information measuring device **characterized by** comprising:

measuring means (10) for continuously measuring biological information of a predetermined site of a human body;
detecting means (320) for detecting a point in time of activity start and a point in time of activity end of the predetermined site on the basis of measurement values measured by the measuring means (10) ;
calculating means (320) for calculating activity efficiency on the basis of an activity time from a point in time of activity start up to a point in time of activity end detected by the detecting means (320), and an activity interval from the point in time of activity start up to a time when a next point in time of activity start is detected by the detecting means (320); and
display control means (320) for controlling a display of the activity efficiency calculated by the calculating means (320).

11. The biological information measuring device according to claim 10, **characterized in that** the detecting means (320) includes comparison means (b3, b6) for comparing measurement values measured by the measuring means (10) with a predetermined threshold value, and detects a point in time when a measurement value exceeds the predetermined threshold value, as a point in time of activity start, and detects a point in time when a measurement value is made less than the predetermined threshold value after the point in time of activity start, as a point in time of activity end.

12. The biological information measuring device according to claim 10, **characterized by** further comprising

counting means (320) for counting a number of activity occurrences at an activity interval,
wherein the display control means (320) controls a display of the activity efficiency calculated by the calculating means (320) and the number of activity occurrences counted by the counting means (320).

13. The biological information measuring device according to claim 10, **characterized in that** the display control means (320) includes change display control means for displaying changes over time in the activity efficiency calculated by the calculating means (320).

14. The biological information measuring device according to claim 10, **characterized in that** the display control means (320) includes average value display control means for controlling a calculation and a display of an average value of the activity efficiency calculated by the calculating means (320).

15. A biological information measuring device **characterized by** comprising:

measuring means (10) for measuring biological information of respective body sites;
condition setting means (310) for setting a measurement condition in the measurement of the biological information by the measuring means (10);
displaying means (330) for displaying measurement results obtained by measuring the biological information by the measuring means (10) with time; and
display cycle setting means (320) for setting a cycle in which the measurement results are displayed on the displaying means (330) in accordance with the measurement condition set by the condition setting means (310).

16. The biological information measuring device according to claim 15, **characterized in that** the measurement condition set by the measurement condition setting means (310) is measurement duration, and the display cycle setting means (320) makes a cycle in which the measurement results are displayed on the displaying means (330) less when the measurement duration is short, and makes the cycle in which the measurement results are displayed on the displaying means (330) greater when the measurement duration is long.

17. A biological information measuring system **characterized by** comprising:

measuring devices (10) which measure biological information of respective body sites; and
a control device (30) which displays measurement results obtained by measuring the biological information by the measuring devices (10) with time,
wherein the control device (30) includes:

condition setting means (310) for setting a measurement condition in the measurement of the biological information by the measuring devices (10);
displaying means (330) for displaying measurement results obtained by measuring the biological information by the measuring devices (10) with time; and
display cycle setting means (320) for setting a cycle in which the measurement results are displayed on the displaying means (330) in accordance with the measurement condition set by the condition setting means (310).

# FIG.1

# FIG.2

MYOELECTRIC POTENTIAL MEASURING DEVICE — 10

MEASURING UNIT — 110

| ELECTRODE UNIT — 111 | IMPEDANCE CONVERSION EQUIPMENT — 112 | AMPLIFIER — 113 | FILTER — 114 | A/D CONVERTER — 115 |

MEMORY UNIT — 140

CPU — 120

TRANSMITTER/RECEIVER UNIT — 130

MYOELECTRIC POTENTIAL MEASURING DEVICE — 10

MEASURING UNIT — 110

| ELECTRODE UNIT — 111 | IMPEDANCE CONVERSION EQUIPMENT — 112 | AMPLIFIER — 113 | FILTER — 114 | A/D CONVERTER — 115 |

MEMORY UNIT — 140

CPU — 120

TRANSMITTER/RECEIVER UNIT — 130

CONTROL UNIT — 30

INPUT UNIT — 310

CPU — 320

DISPLAY UNIT — 330

MEMORY UNIT — 350

TRANSMITTER/RECEIVER UNIT — 340

15

# FIG.3A

# FIG.3B

# FIG.4

**140**

MEMORY UNIT

| |
|---|
| MUSCLE STATE MEASUREMENT PROGRAM — **141** |
| MYOELECTRIC POTENTIAL GENERATION DATA — **142** |
| MUSCLE ACTIVITY START TIMING — **143** |
| MUSCLE ACTIVITY END TIMING — **144** |
| MUSCULAR STRENGTH KEEPING DURATION (Tv) — **145** |
| MYOELECTRIC POTENTIAL GENERATION INTERVAL (Tsyc) — **146** |
| NUMBER OF PEDALING — **147** |
| AVERAGED RECTIFIED VALUE (ARV) — **148** |
| MEAN POWER FREQUENCY (MPF) — **149** |

# FIG.5

**350**

MEMORY UNIT

| |
|---|
| MUSCLE STATE DISPLAY PROGRAM — **351** |
| MUSCLE STATE DATA (RIGHT LEG) |
| MUSCLE STATE DATA (LEFT LEG) — **353** |
| PEDALING EFFICIENCY DATA (RIGHT LEG) |
| PEDALING EFFICIENCY DATA (LEFT LEG) — **355** |
| AVERAGE PEDALING EFFICIENCY (RIGHT LEG) |
| AVERAGE PEDALING EFFICIENCY (LEFT LEG) — **357** |

# FIG.6

| NUMBER OF PEDALING | MUSCULAR STRENGTH KEEPING DURATION (Tv) | MYOELECTRIC POTENTIAL GENERATING INTERVAL (Tsyc) | AVERAGED RECTIFIED VALUE (ARV) | MEAN POWER FREQUENCY (MPF) |
|---|---|---|---|---|
| 1 | Tv1 | Tsyc1 | ARV1 | MPF1 |
| 2 | Tv2 | Tsyc2 | ARV2 | MPF2 |
| 3 | Tv3 | Tsyc3 | ARV3 | MPF3 |
| ⋮ | ⋮ | ⋮ | ⋮ | ⋮ |

EP 1 839 572 A2

# FIG.7A

# FIG.7B

# FIG.8A

# FIG.8B

# EP 1 839 572 A2

# FIG.9

**MUSCLE STATE DISPLAY PROCESS IN CONTROL UNIT**

START

a1 — INPUT EXERCISE MODE

a2 — IS EXERCISE MODE SHORT-TERM MODE? → YES → a3 — SET DISPLAY INTERVAL TO TEN SECONDS

NO

a4 — IS EXERCISE MODE MEDIUM-TERM MODE? → YES → a5 — SET DISPLAY INTERVAL TO ONE MINUTE

NO

a6 — IS EXERCISE MODE LONG-TERM MODE? → NO

YES

a7 — SET DISPLAY INTERVAL TO FIVE MINUTES

a8 — INPUT MEASUREMENT START INSTRUCTION

a9 — TRANSMIT EXERCISE MODE INSTRUCTION SIGNAL AND MEASUREMENT START SIGNAL TO EACH OF MYOELECTRIC POTENTIAL MEASURING DEVICES

(1)

**MUSCLE STATE MEASUREMENT PROCESS IN MYOELECTRIC POTENTIAL MEASURING DEVICE**

START

b1 — RECEIVE EXERCISE MODE INSTRUCTION SIGNAL AND MEASUREMENT START SIGNAL

b2 — START MEASURING MYOELECTRIC POTENTIALS

b3 — IS VALUE OF MEASURED MYOELECTRIC POTENTIAL GREATER THAN OR EQUAL TO REFERENCE VALUE? → NO

YES ← (3)

b4 — DETERMINE POINT IN TIME WHEN IT IS JUDGED THAT VALUE IS GREATER THAN OR EQUAL TO REFERENCE VALUE, AS MUSCLE ACTIVITY START TIMING (Ta)

b5 — START STORING VALUES OF MYOELECTRIC POTENTIALS

b6 — IS VALUE OF MEASURED MYOELECTRIC POTENTIAL LESS THAN REFERENCE VALUE? → NO

YES

b7 — HAS MUSCLE ACTIVITY BEEN TERMINATED? → NO

YES

(2)

# FIG.10

(2)

**b8** — DETERMINE POINT IN TIME WHEN VALUE IS MADE LESS THAN REFERENCE VALUE, AS MUSCLE ACTIVITY END TIMING (tb)

**b9** — CALCULATE MUSCLE STRENGTH KEEPING DURATION (Tv)

**b10** — IS MEASURED MYOELECTRIC POTENTIAL GREATER THAN OR EQUAL TO REFERENCE VALUE?

NO

YES → (3)

**b11** — TERMINATE STORAGE OF VALUES OF MYOELECTRIC POTENTIALS

**b12** — UPDATE NUMBER OF PEDALING

**b13** — CALCULATE MYOELECTRIC POTENTIAL GENERATING INTERVAL (Tsyc)

**b14** — CALCULATE AVERAGED RECTIFIED VALUE (ARV) AND MEAN POWER FREQUENCY (MPF)

**b15** — TRANSMIT MEASUREMENT RESULTS

**a10** — RECEIVE MEASUREMENT RESULTS

**a11** — UPDATE CORRESPONDING MUSCLE STATE DATA

**a12** — CALCULATE PEDALING EFFICIENCY

**a13** — CALCULATE AVERAGE PEDALING EFFICIENCY

**a14** — DISPLAY MUSCLE STATE DATA, PEDALING EFFICIENCY, AND AVERAGE PEDALING EFFICIENCY ON DISPLAY UNIT

**a15** — HAS SET DISPLAY INTERVAL PASSED?

NO

YES

**a16** — HAS FOLLOWING MEASUREMENT RESULT BEEN RECEIVED?

YES

NO

END

21

**FIG.11A**

330

BEFORE 100sec. / BEFORE 90sec. / BEFORE 80sec. / BEFORE 70sec. / BEFORE 60sec. / BEFORE 50sec. / BEFORE 40sec. / BEFORE 30sec. / BEFORE 20sec. / BEFORE 10sec.

**FIG.11B**

330

BEFORE 10min. / BEFORE 9min. / BEFORE 8min. / BEFORE 7min. / BEFORE 6min. / BEFORE 5min. / BEFORE 4min. / BEFORE 3min. / BEFORE 2min. / BEFORE 1min.

**FIG.11C**

330

BEFORE 50min. / BEFORE 45min. / BEFORE 40min. / BEFORE 35min. / BEFORE 30min. / BEFORE 25min. / BEFORE 20min. / BEFORE 15min. / BEFORE 10min. / BEFORE 5min.

**EP 1 839 572 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004202196 A